# EUROPEAN PATENT APPLICATION

(11) **EP 3 949 998 A1**
(43) Date of publication of application: **09.02.2022**
(21) Application number: 20783648.7
(22) Date of filing: 01.04.2020
(51) Int. Cl.: A61K 45/00, A61P 43/00, A61P 17/00, A61K 36/02, A61K 8/27, A61K 8/29, A61K 8/49, A61K 8/96, A61K 33/24, A61K 33/30, A61K 31/409, A61K 35/748, A61K 41/00

(54) **CELL ACTIVATOR**

(30) Priority: 05.04.2019 JP 2019072750; 31.01.2020 WO PCT/JP2020/003810
(71) Applicant: Shiseido Company, Ltd., Chuo-ku Tokyo 104-0061 (JP)
(72) Inventor: MIYAZAWA, Kazuyuki, Tokyo 104-0061 (JP); GILLET, Renaud, Tokyo 104-0061 (JP); MCCARTHY, Bianca, Tokyo 104-0061 (JP); KANEMARU, Tetsuya, Tokyo 104-0061 (JP)
(74) Representative: Cabinet Beau de Loménie
(86) International application number: PCT/JP2020/015108
(87) International publication number: WO 2020/204105

(57) **Abstract**

[Problem] To provide a novel cell activator.

[Solution] Provided are: a cell activator comprising a wavelength conversion substance as an active ingredient; a composition and a product comprising the cell activator; and a method for activating skin using thereof. The present invention can exhibit a desirable effect on skin by effectively making use of ultraviolet light to activate skin cells.

## Description

### FIELD

The present invention relates to a cell activator comprising a wavelength conversion substance, to a composition and product comprising the cell activator, and to a method for activating skin using the same.

### BACKGROUND

The harm to skin caused by ultraviolet light includes adverse effects such as skin cancer, photoaging, skin spots, wrinkles and inflammation, which are also undesirable from the viewpoint of health and beauty. While ultraviolet light is also utilized for sterilizing purposes, when considered in balance with the harm caused by ultraviolet light there is a greater need to defend against rather than to actively utilize ultraviolet light.

Many measures are therefore being taken to protect the skin from ultraviolet light. Such measures include the use of sunscreens, the implementation of indoor spaces for avoidance of sunlight, and the use of head coverings and clothing treated to block UV rays and films designed to block UV rays.

### [CITATION LIST]

### [PATENT LITERATURE]

[PTL 1] Japanese Patent Publication No. 6424656
[PTL 2] Japanese Patent Publication No. 6361416
[PTL 3] International Patent Publication No. 2018/004006
[PTL 4] Japanese Unexamined Patent Publication No. 2018-131422
[PTL 5] Japanese Unexamined Patent Publication HEI No. 5-117127
[PTL 6] Japanese Patent Publication No. 4048420
[PTL 7] Japanese Patent Publication No. 4677250
[PTL 8] Japanese Patent Publication No. 3303942
[PTL 9] Japanese Unexamined Patent Publication No. 2017-88719
[PTL 10] International Patent Publication No. 2018/117117

### SUMMARY

### [TECHNICAL PROBLEM]

It is an object of the present invention to provide a novel cell activator that utilizes ultraviolet light.

### [SOLUTION TO PROBLEM]

The present inventors have conducted active research with the aim of allowing ultraviolet light to be effectively utilized on skin. As a result, a cell activator has been devised that converts ultraviolet light wavelengths and activates skin cells.

The present application provides the present invention with the aspects set forth below.
(1) A cell activator comprising a wavelength conversion substance as an active ingredient, wherein the wavelength conversion substance converts the wavelength of ultraviolet light contained in incident light to emit emission light having a wavelength longer than the wavelength of the ultraviolet light.
(2) The cell activator according to (1), wherein the ultraviolet light has a peak wavelength between 200 nm and 400 nm.
(3) The cell activator according to (1) or (2), wherein the emission light has a peak wavelength between 500 nm and 700 nm.
(4) The cell activator according to any one of (1) to (3), wherein the wavelength conversion substance comprises one or more phycobiliproteins selected from among allophycocyanin, C-phycocyanin, R-phycocyanin, phycoerythrocyanin, B-phycoerythrin, b-phycoerythrin, C-phycoerythrin and R-phycoerythrin; one or more inorganic phosphors selected from among zinc oxide phosphors, magnesium titanate phosphors and calcium phosphate phosphors; one or more components selected from among vitamin A, β-carotene, vitamin K, vitamin B1, vitamin B2, vitamin B6, vitamin B12, folic acid, niacin, lycopene, gardenia, safflower, turmeric, cochineal, perilla, red cabbage, flavonoids, carotenoids, quinoids, porphyrins, anthocyanins, polyphenols, salicylic acid and capsicum extract; and/or one or more pigments selected from among Red No. 401, Red No. 227, Red No. 504, Red No. 218, Orange No. 205P, Yellow No. 4, Yellow No. 5, Green No. 201, Pyranin Conch, Blue No. 1, 2,4-diaminophenoxyethanol hydrochloride, Arizulin Purple SS, Violet No. 401, Black No. 401, Helindone Pink, Yellow No. 401, Bentizine Yellow G, Blue No. 404, Red No. 104, meta-aminophenol, capsicum pigment, gardenia pigment and paprika pigment.
(5) The cell activator according to (4), wherein the wavelength conversion substance comprises one or more phycobiliproteins selected from among allophycocyanin, C-phycocyanin, R-phycocyanin, phycoerythrocyanin, B-phycoerythrin, b-phycoerythrin, C-phycoerythrin and R-phycoerythrin; one or more inorganic phosphors selected from among zinc oxide phosphors, magnesium titanate phosphors and calcium phosphate phosphors; and/or one or more B vitamins selected from among vitamin B1, vitamin B2, vitamin B6 and vitamin B12.
(6) A composition comprising the cell activator according to any one of (1) to (5).
(7) The composition according to (6), wherein the composition is a skin external composition for activating skin by exposing skin to light containing ultraviolet light.
(8) A cosmetic method for activating skin of a subject, comprising:
   applying the composition according to (6) or (7) to skin of a subject, and
   exposing the composition-applied skin with light containing ultraviolet light.
(9) A product comprising the cell activator according to any one of (1) to (5).
(10) The product according to (9), wherein the product is for activating skin by exposing the skin to light passing through the product, which contains ultraviolet light.
(11) A cosmetic method for activating skin of a subject, comprising:
   bringing light containing ultraviolet light through the product according to (9) or (10), and
   exposing the skin of the subject to light passing through the product.

### [ADVANTAGEOUS EFFECTS OF INVENTION]

The present invention can exhibit a desirable effect on skin by effectively making use of ultraviolet light to activate skin cells. Because it is conventionally undesirable for skin to be exposed to ultraviolet light, it has been common technical knowledge in the field to apply measures to maximally avoid exposing skin to ultraviolet light. It was highly surprising to find, then, that the present invention provides a desirable effect on skin by utilization of ultraviolet light. The invention provides novel uses of the aforementioned compounds that have conventionally been used primarily as dyes, pigments, ultraviolet scattering agents, ultraviolet absorbers, nutrients and antioxidants. The invention also helps to improve quality of life by providing a more positive feeling for persons who have attempted to avoid ultraviolet light as much as possible for beauty or health reasons when outdoors.

### BRIEF DESCRIPTION OF DRAWINGS

Fig. 1 is a schematic diagram illustrating Experiments 1 and 2.
Fig. 2 shows cellular activity during irradiation of UV using different wavelength conversion substances in Experiment 1. The ordinate represents relative fluorescence intensity (au).
Fig. 3 shows cellular activity during irradiation of strong UV using C-phycocyanin at different concentrations in Experiment 2, in terms of relative fluorescence intensity (au).
Fig. 4 is a schematic diagram illustrating Experiment 3.
Fig. 5 shows cellular activity during irradiation of UV using C-phycocyanin in cells with previously lowered cellular activity in Experiment 3, in terms of relative fluorescence intensity (au) (P test).
Fig. 6 shows cellular activity during irradiation of UV using a zinc oxide phosphor in cells with previously lowered cellular activity in Experiment 4, in terms of relative fluorescence intensity (au) (P test).

### DESCRIPTION OF EMBODIMENTS

The cell activator of the invention comprises a wavelength conversion substance as an active ingredient. A wavelength conversion substance is a substance that converts the wavelength of ultraviolet light contained in incident light and emits emission light having a wavelength longer than the wavelength of the ultraviolet light.

The ultraviolet light may include UVA, UVB and UVC. According to one embodiment, the ultraviolet light is light with a peak wavelength of 200 nm to 400 nm. The ultraviolet light may also be included in incident light such as sunlight, for example. Alternatively, the incident light may be ultraviolet light, and artificially generated ultraviolet light may be used.

The emission light emitted by the wavelength conversion substance has a longer wavelength than ultraviolet light, with a peak wavelength of preferably 500 nm to 700 nm. The emission light may have one or more peaks at 510 nm, 520 nm, 530 nm, 540 nm, 550 nm, 560 nm, 570 nm, 580 nm, 590 nm, 600 nm, 610 nm, 620 nm, 630 nm, 640 nm, 650 nm, 660 nm, 670 nm, 680 nm, 690 nm or 700 nm, or in any range within these values, though without being restrictive, or it may be red light, orange light, green light or blue light. According to one embodiment, the wavelength conversion substance has its main wavelength at 500 nm to 700 nm, for light emitted upon excitation with excitation light of 200 nm to 400 nm.

Examples of wavelength conversion substances include the following components: phycobiliproteins such as allophycocyanin, C-phycocyanin, R-phycocyanin, phycoerythrocyanin, B-phycoerythrin, b-phycoerythrin, C-phycoerythrin and R-phycoerythrin; natural or synthetic components such as vitamin A, β-carotene, vitamin K, vitamin B1, vitamin B2, vitamin B6, vitamin B12, folic acid, niacin, lycopene, gardenia, safflower, turmeric, cochineal, perilla, red cabbage, flavonoids, carotenoids, quinoids, porphyrins, anthocyanins, polyphenols, salicylic acid and capsicum extract; dyes such as Red No. 401, Red No. 227, Red No. 504, Red No. 218, Orange No. 205 P, Yellow No. 4, Yellow No. 5, Green No. 201, Pyranin Conch, Blue No. 1, 2,4-diaminophenoxyethanol hydrochloride, Arizulin Purple SS, Violet No. 401, Black No. 401, Helindone Pink, Yellow No. 401, Bentizine Yellow G, Blue No. 404, Red No. 104, *meta-*aminophenol, capsicum pigment, gardenia pigment and paprika pigment; and phosphors obtained by fluorescent-doping of inorganic compounds, for example, blue phosphors comprising the amorphous silica particles mentioned in Japanese Patent No. 6424656, cerium and phosphorus and/or magnesium, and red phosphors comprising compounds obtained by europium activation of mixed crystals consisting of the alkaline earth metal sulfides described in Japanese Patent No. 6361416 combined with gallium compounds, the zinc oxide phosphors mentioned in International Patent Publication No. 2018/004006, the zinc oxide phosphors mentioned in Japanese Unexamined Patent Publication No. 2018-131422, and the inorganic phosphors mentioned in Japanese Unexamined Patent Publication HEI No. 5-117127. According to one embodiment, the inorganic phosphor is one or more phosphors selected from among phosphors obtained by doping zinc oxides represented by ZnO: Zn, Zn_{1+z}, ZnO₁₋ₓ with the sulfur-containing compounds mentioned in International Patent Publication No. 2018/004006, including sulfides and/or sulfates such as zinc sulfide or zinc sulfate, magnesium titanate phosphors obtained by doping magnesium titanates such as MgTiO₃ or Mg₂TiO₄ with manganese, and calcium phosphate phosphors obtained by doping calcium phosphates such as Ca(H₂PO₄)₂, CaHPO₄ or Ca₃(PO₄)₂ with cerium.

The wavelength conversion substance may be obtained by extraction from a natural source such as an animal, plant or algae, or it may be obtained by an artificial method such as chemical synthesis. For example, phycobiliproteins can be prepared by extraction from algae, including blue-green algae such as spirulina *(Spirulina platensis)* or red algae such as porphyridiophylla *(Porphyridium purpureum),* by the method described in Japanese Patent No. 4048420, Japanese Patent No. 4677250 or Japanese Patent No. 3303942, for example. Zinc oxide phosphors can be produced by the method described in International Patent Publication No. 2018/004006, Japanese Unexamined Patent Publication No. 2018-131422 or Japanese Unexamined Patent Publication HEI No. 5-117127, for example. Magnesium titanate phosphors can be produced by the method described in Japanese Unexamined Patent Publication No. 2017-88719. Calcium phosphate phosphors can be produced by the method described in International Patent Publication No. 2018/117117.

So long as the wavelength conversion effect of the invention is not impaired, these wavelength conversion substances may be composed of, or may include, the components mentioned above, and they may be single components alone or combinations of more than one of the components. For example, the aforementioned phycobiliproteins or inorganic material phosphors may be mixed with other wavelength conversion substances such as B vitamins (vitamin B1, vitamin B2, vitamin B6 or vitamin B12) to exhibit synergistic effects. These components are merely examples, however, and any other substances that exhibit the wavelength conversion effect of the invention may be used.

The wavelength conversion substance content in the cell activator, composition or product of the invention is not particularly restricted so long as the wavelength conversion effect of the invention is not impaired, and it may be appropriately determined for the type of wavelength conversion substance and the purpose of use of the cell activator or composition. It may be any content in the range of 0.01 to 99.99 wt% or 0.1% to 999 wt%, for example.

The cell activation is not restricted but may be accelerated metabolism or turnover of cells of an animal such as a human, such as skin fibroblasts and/or keratinocytes, or improvement of their function, or accelerated proliferation, inhibited oxidation, improved resistance against fatigue or external stimulation, suppressed reduction in function or activity, or cellular repair. Activation of skin cells is expected to provide an effect of preventing or ameliorating wrinkles, blemishes, skin aging and photoaging.

Measurement of the cell activation effect may be carried out by measuring cell viability or proliferation using Alamar Blue, as in the Examples, or other methods such as dye assays, mitochondrial membrane potential-dependent dye assay, elastase cleavage dye assay, ATP and ADE assay, glycolysis flux and oxygen consumption assay.

Any form of administration may be used for the cell activator and composition of the invention, but an external preparation for skin will often be preferred, such as a drug, quasi drug or cosmetic, for activating skin by exposing the skin to light containing ultraviolet light. When the cell activator or composition of the invention is to be used as an external preparation for skin, the dosage form, coating method and number of doses may be determined as desired. For example, it may be applied onto skin in the form of cosmetic water or a spray, oil, cream, latex, gel, sunscreen or suntan lotion either periodically or irregularly, once or several times per day at morning, noon or evening, or before going out or engaging in outdoor activities, marine sports or skiing, for example, when exposure to sunlight is expected.

The cell activator and composition of the invention may also be used in combination with an additive such as an excipient, preservative, thickener, binder, disintegrator, dispersing agent, stabilizer, gelling agent, antioxidant, surfactant, preservative, oil, powder, water, alcohol, thickener, chelating agent, silicone, antioxidant, humectant, aromatic, drug component, antiseptic agent, pH adjustor or neutralizer, selected as necessary or desired. It may also be used in combination with other cell activators to increase the effect of the invention.

The present invention further provides products such as sun visors, caps, clothing, gloves, screen films, window sprays or creams, window materials or wall materials, for example, that comprise a cell activator of the invention and are intended to activate skin by exposing the skin to light containing ultraviolet light. The usage of additives in the products of the invention and the forms of the products may also be as desired.

The present invention further provides a method for producing the cell activator, composition or product of the invention. A method for activating skin of a subject is also provided, the method comprising application of a cell activator or composition of the invention onto the skin of a subject and exposing the skin to light containing ultraviolet light after application of the cell activator or composition; or passing light containing ultraviolet light through the product of the invention, and exposing the skin to the transmitted light; wherein the cell activator, composition or product converts the wavelength of ultraviolet light in the incident light and emits emission light with a longer wavelength than the wavelength of the ultraviolet light, transmitting the ultraviolet light with a peak wavelength of preferably 200 nm to 400 nm as light with a peak wavelength of 500 nm to 700 nm. The method for activating skin will often be for the purpose of beautifying, instead of treatment by a doctor or health care professional. The invention further provides a cosmetic counseling method for supporting cosmetology, which includes providing a cosmetic method, cell activator, composition or product of the invention to a subject.

### EXAMPLES

The present invention will now be explained in greater detail by examples. However, the invention is in no way limited by the examples.

Experiment 1: Cell activation effects of wavelength conversion substances
Experiment 1-1: Preparation of wavelength conversion substances Wavelength conversion substances were prepared in the following manner.

### (1) B-phycoerythrin

B-phycoerythrin is obtained from porphyridiophylla *(Porphiridium cruentum)* extract, the absorption spectrum having a peak wavelength at 305 nm and the emission spectrum having peak wavelengths at 570 nm and 610 nm.

### (2) C-phycocyanin

C-phycocyanin is obtained from spirulina *(Spirulina platensis)* extract, the absorption spectrum having a peak wavelength at 350 nm and the emission spectrum having peak wavelengths at 640 nm and 700 nm.

### (3) Zinc oxide phosphor

Lumate G by Sakai Chemical Industry Co., Ltd. was used. Lumate G is a zinc oxide phosphor obtained by doping ZnO with a sulfur-containing compound and then firing as described in International Patent Publication No. 2018/004006, the absorption spectrum having a peak wavelength at 365 nm and the emission spectrum having a peak wavelength at 510 nm. (4) Magnesium titanate phosphor

Lumate R by Sakai Chemical Industry Co., Ltd. was used. Lumate R is a magnesium titanate phosphor obtained by doping MgTiO₃ with manganese, the absorption spectrum having a peak wavelength at 365 nm and the emission spectrum having peak wavelengths in the range of 660 to 680 nm.

The wavelength conversion substances of (1) and (2) were dissolved in water to prepare solutions at concentrations of 1% and 5%.

The wavelength conversion substances of (3) and (4) were dispersed in alcohol to prepare 5% and 10% dispersions.

### Experiment 1-2: Preparation of cell samples

Cell samples were prepared in the following manner.
1. Human skin fibroblasts (purchased from Kurabo) and human skin keratinocytes (purchased from Kurabo or PromoCell) were used. A cell suspension (1 mL) stored with liquid nitrogen was placed in a hot water bath (37°C) and thawed until small ice pellets remained, and then diluted with 9 mL of warm medium.
2. The diluted suspension was gently mixed and transferred to a T75 flask, and incubated overnight at 37°C.
3. On the following day, the medium was exchanged with 10 mL of fresh medium.
4. The medium was periodically exchanged (once every 2 days for fibroblasts, or once every 2 to 3 days for keratinocytes), while continuing growth of the cells. During this time, a microscope was used to observe the cells and to confirm that the cells had proliferated with the proper form.
5. Once the cells reached approximately 80% confluence, they were subcultured. Subculturing of the cells was carried out by rinsing once in 10 mL of warm PBS, after which 5 mL of warm trypsin was added to the T75 flask to cover the bottom of the flask with the trypsin solution, and the mixture was suctioned after standing for 1 minute at room temperature.
6. The flask was set in an oven at 37°C for 2 minutes (maximum) for fibroblasts and 7 minutes (maximum) for keratinocytes. A microscope was used to observe the cells, confirming that they were small and elliptical.
7. The sides of the T75 flask were then lightly tapped to free the cells. A microscope was used to observe the cells and confirm that they were freely moving.
8. The fibroblasts were resuspended in 5 mL of warm FGM (containing 10% serum), sterilized and transferred to a 50 mL Falcon tube. The flask was further rinsed with 5 mL of warm FGM and added to the Falcon tube, to ensure transfer of all of the cells.
9. The cells were centrifuged at 10,000 rpm for 5 minutes (4°C), and the supernatant was carefully removed while avoiding disturbing the cell pellet.
10. Depending on their type, the cells were resuspended in FGM or KGM at a concentration of 2 × 10⁴ cells/well (500 µL) for fibroblasts or 4 × 10⁴ cells/well (500 µL) for keratinocytes, and plated in a 24-well plate.
11. The cells were seeded in the 24-well plate, with periodic exchange of the medium (once every 2 days for fibroblasts, or once every 2 to 3 days for keratinocytes), growing the cells until they reached 60 to 70% confluence (differing for the type of experiment). (Note: fibroblasts are expected to reach desired confluence by 24 hours assuming a cell density of 2 × 10⁴ cells/well. When the cell density is as low as 1 × 10⁴ cells/well, for example, fibroblasts require 48 hours to reach desired confluence.)
12. At 24 hours before irradiation, the medium was changed to non-supplemented medium (for keratinocytes) or low-concentration serum-containing (0.5% FCS) medium (for fibroblasts).

### Experiment 1-3: Ultraviolet light irradiation

1. At least 30 minutes prior to irradiation, the power source of a solar simulator was activated to warm up the lamp. The solar simulator used was a UG11 filter. A UG11 filter is a filter that allows passage of UVB alone while cutting light of other wavelengths. The UV light passing through the UG11 filter had a peak wavelength of 300 nm to 385 nm.
2. The temperature control plate was turned on and set to 33°C.
3. The cells prepared in Experiment 1-2 were rinsed once with warm PBS.
4. A 0.5 mL portion of warmed Martinez solution (145 mM NaCl, 5.5 mM KCl, 1.2 mM MgCl₂·6H₂O, 1.2 mM NaH₂PO₄·2H₂O, 7.5 mM HEPES, 1 mM CaCl₂ and 10 mM D-glucose) was added to each well.
5. As shown in Fig. 1, the cell-containing wells were set on a plate, 0.4 ml of each solution containing the wavelength conversion substances (1) to (4) prepared in Experiment 1-1 was injected into the wells of a 24-well plate, the cell-containing wells were placed over it in a manner covering the wells, and UV light was irradiated into the cell solution through the wavelength conversion substance solution without allowing direct contact between the wavelength conversion substance solution and the cell solution.
6. The irradiation was carried out to a total radiation dose of 100 mJ/cm². As controls, there were prepared a sample of the cells directly irradiated with UV light without setting a wavelength conversion substance plate on the cell-containing wells, and a sample of the cells cultured in a dark environment without irradiation of UV light.
7. After irradiation, the Martinez was exchanged with warmed KGM (supplement-free) or FGM (0.5% FCS), and the plate was returned to the 37°C incubator.

### Experiment 1-4: Measurement of cellular activity

The cells kept at 48 hours in an incubator after Experiment 1-3 were used for activity measurement by the following method.
1. To medium (supplement-free KGM medium or 0.5% FCS-containing FGM medium) there was added 10% Alamar Blue, and the mixture was heated to 37°C (with the solution kept in a dark environment).
2. The medium in the wells was exchanged with 500 µL of the 10% Alamar Blue solution, and the plate was returned to the 37°C incubator and kept for about 3 hours. The control wells were also kept in the incubator. The solution was kept in a dark environment to protect it from light.
3. After 3 hours, a 100 µL aliquot was harvested and transferred to a black 96-well plate.
4. A fluorometer (OPwave+, Ocean Photonics) was used to read off the measured fluorescence value at 544 nm/590 nm.

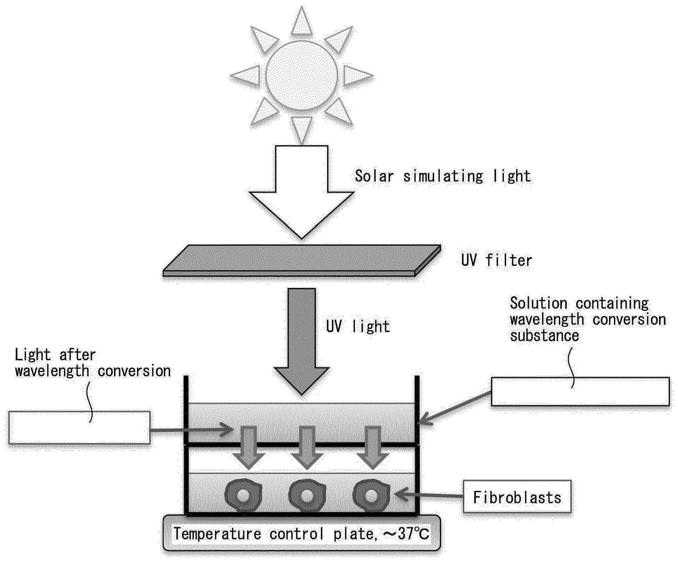

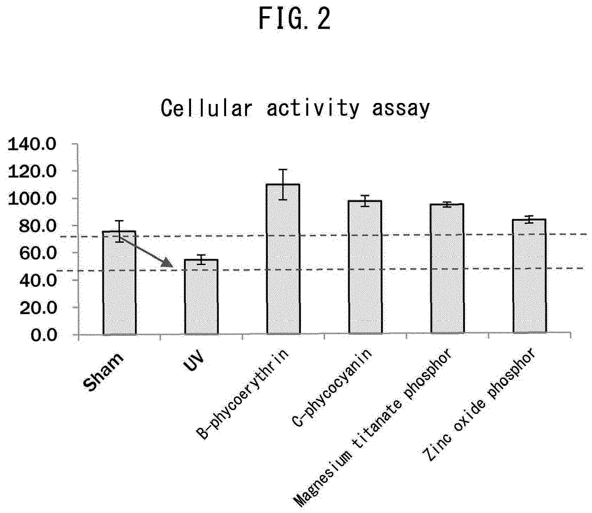

The results are shown in Fig. 2. UV irradiation lowered the cellular activity compared to the non-irradiated control. However, the activity of the cells irradiated with UV through the wavelength conversion substances was higher compared to non-irradiation, for all of the wavelength conversion substances. These results demonstrated that cellular activity is reduced with UV irradiation, but that using a wavelength conversion substance inhibits the reduction in cellular activity.

### Experiment 2: Effects of different wavelength conversion substance concentrations and UV strengths on cellular activity

The same method was used as in Experiment 1, except that C-phycocyanin was used as the wavelength conversion substance, the cell cultures were covered with plates containing solutions with addition at 0%, 0.4% and 2%, and the UV was irradiated at doses of 0, 10, 25, 50, 75 and 100 mJ/cm^{2.}

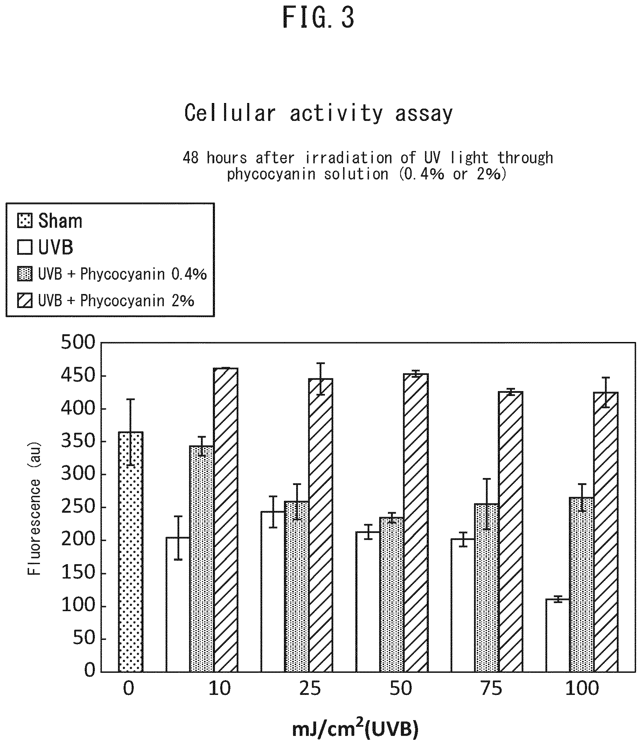

The results are shown in Fig. 3. When no wavelength conversion substance was used, cellular activity was reduced with increasing UV irradiation dose. With addition of 0.4% C-phycocyanin, however, reduction in cellular activity was inhibited even under UV irradiation, while addition of 2% C-phycocyanin actually accelerated cellular activity even above the level with no UV irradiation. These results demonstrated that, while cellular activity is reduced by UV irradiation, using a wavelength conversion substance not only inhibits reduction in cellular activity in a concentration-dependent manner but even accelerates cellular activity.

### Experiment 3: Restoration of UV irradiation-reduced cellular activity by C-phycocyanin

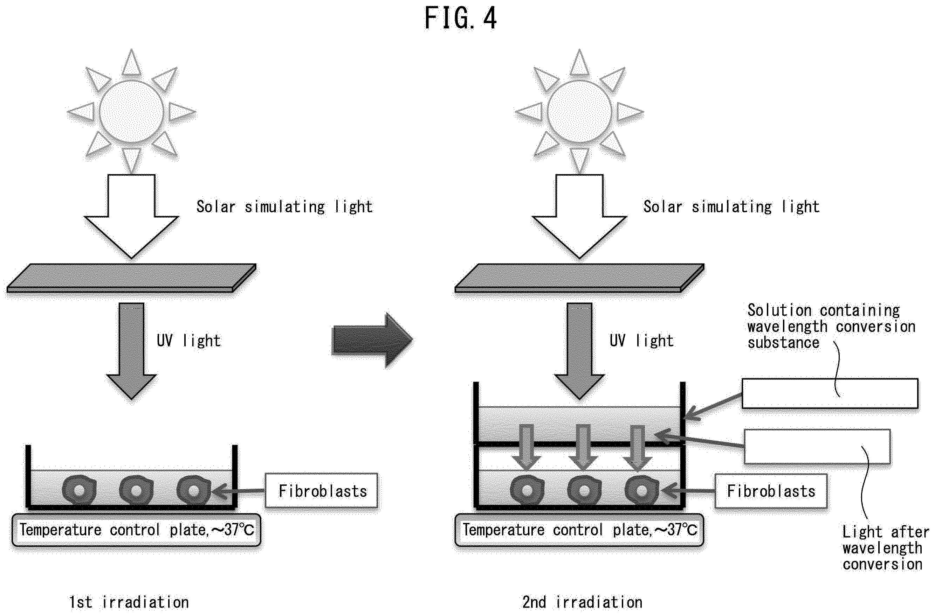

The same method was used as in Experiment 1, except that after cellular activity was first lowered by UV irradiation with an exposure dose of 400 mJ/cm² without using a wavelength conversion substance, as shown in Fig. 4, the cell cultures were covered with a solutioncontaining plate with C-phycocyanin added as the wavelength conversion substance at 0%, 0.4% and 2%, and UV irradiation was with radiation doses of 0, 10, 25, 50, 75, 100 and 200 mJ/cm^{2.}

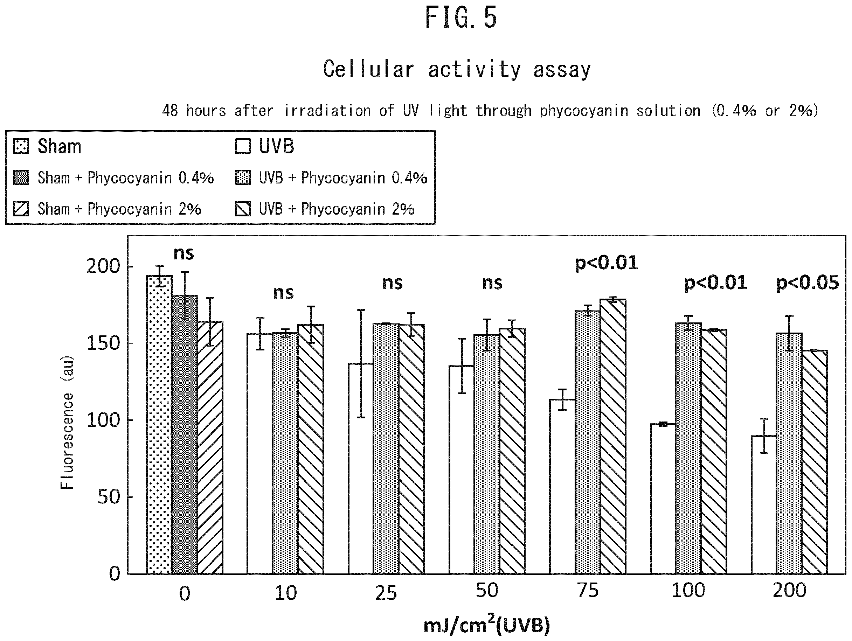

The results are shown in Fig. 5. It is seen that cellular activity was restored by UV irradiation using a wavelength conversion substance, even in the cells that had previously reduced activity by UV irradiation without using a wavelength conversion substance. The effect with 0.4% concentration of C-phycocyanin was similar to the effect with 2%, suggesting that an adequate cell activation effect is obtained even at 0.4%. When UV irradiation was carried out without using a wavelength conversion substance, on the other hand, the cellular activity was reduced in a manner dependent on the UV radiation dose.

Fig. 2, Fig. 3 and Fig. 5 show the experimental results for human skin fibroblasts, but similar results were observed for keratinocytes as well. In the next experiment, keratinocytes obtained by the method of Experiment 1-2 were used to examine the cell activation effect when using a film comprising a zinc oxide phosphor as the wavelength conversion substance.

### Experiment 4: Restoration of UV irradiation-lowered cellular activity by zinc oxide phosphor

The same method was used as in Experiment 3, except that after the cellular activity of human skin keratinocytes was first lowered by UV irradiation with an exposure dose of 400 mJ/cm² without using a wavelength conversion substance, UV irradiation was carried out with a radiation dose of 100 mJ/cm², either with the cell culture covered with a film prepared by dispersing a zinc oxide phosphor as a wavelength conversion substance in an acrylic polymer to 5%, or without using a film.

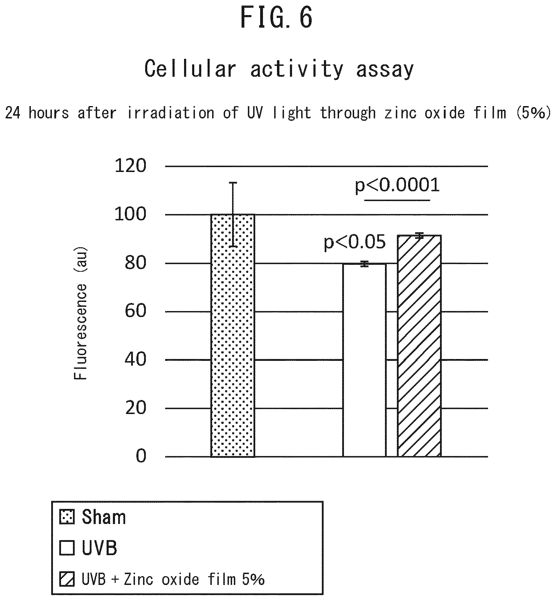

The results are shown in Fig. 6. It is seen that even when keratinocytes were used, similar to Experiment 3, the cellular activity of the cells that had been previously lowered by UV irradiation was restored by the wavelength conversion substance, when the film comprising a zinc oxide phosphor as the wavelength conversion substance was used.

These results demonstrated that a wavelength conversion substance has an effect of not only inhibiting reduction in cellular activity by UV irradiation, but also of activating cells utilizing UV light. Activation of skin cells is expected to prevent or ameliorate wrinkles, blemishes, skin aging and photoaging.

The embodiments of the invention described above are not intended to place limitations on the invention, and various modifications including cosmetics and drug compositions may be incorporated, which fall within the gist of the invention.

## Claims

1. A cell activator comprising a wavelength conversion substance as an active ingredient, wherein the wavelength conversion substance converts the wavelength of ultraviolet light contained in incident light to emit emission light having a wavelength longer than the wavelength of the ultraviolet light.

2. The cell activator according to claim 1, wherein the ultraviolet light has a peak wavelength between 200 nm and 400 nm.

3. The cell activator according to claim 1 or 2, wherein the emission light has a peak wavelength between 500 nm and 700 nm.

4. The cell activator according to any one of claims 1 to 3, wherein the wavelength conversion substance comprises one or more phycobiliproteins selected from among allophycocyanin, C-phycocyanin, R-phycocyanin, phycoerythrocyanin, B-phycoerythrin, b-phycoerythrin, C-phycoerythrin and R-phycoerythrin; one or more inorganic phosphors selected from among zinc oxide phosphors, magnesium titanate phosphors and calcium phosphate phosphors; one or more components selected from among vitamin A, β-carotene, vitamin K, vitamin B1, vitamin B2, vitamin B6, vitamin B12, folic acid, niacin, lycopene, gardenia, safflower, turmeric, cochineal, perilla, red cabbage, flavonoids, carotenoids, quinoids, porphyrins, anthocyanins, polyphenols, salicylic acid and capsicum extract; and/or one or more pigments selected from among Red No. 401, Red No. 227, Red No. 504, Red No. 218, Orange No. 205P, Yellow No. 4, Yellow No. 5, Green No. 201, Pyranin Conch, Blue No. 1, 2,4-diaminophenoxyethanol hydrochloride, Arizulin Purple SS, Violet No. 401, Black No. 401, Helindone Pink, Yellow No. 401, Bentizine Yellow G, Blue No. 404, Red No. 104, meta-aminophenol, capsicum pigment, gardenia pigment and paprika pigment.

5. The cell activator according to claim 4, wherein the wavelength conversion substance comprises one or more phycobiliproteins selected from among allophycocyanin, C-phycocyanin, R-phycocyanin, phycoerythrocyanin, B-phycoerythrin, b-phycoerythrin, C-phycoerythrin and R-phycoerythrin; one or more inorganic phosphors selected from among zinc oxide phosphors, magnesium titanate phosphors and calcium phosphate phosphors; and/or one or more B vitamins selected from among vitamin B1, vitamin B2, vitamin B6 and vitamin B12.

6. A composition comprising the cell activator according to any one of claims 1 to 5.

7. The composition according to claim 6, wherein the composition is a skin external composition for activating skin by exposing skin to light containing ultraviolet light.

8. A cosmetic method for activating skin of a subject, comprising:
applying the composition according to claim 6 or 7 to skin of a subject, and
exposing the composition-applied skin with light containing ultraviolet light.

9. A product comprising the cell activator according to any one of claims 1 to 5.

10. The product according to claim 9, wherein the product is for activating skin by exposing the skin to light passing through the product, which contains ultraviolet light.

11. A cosmetic method for activating skin of a subject, comprising:
bringing light containing ultraviolet light through the product according to claim 9 or 10, and
exposing the skin of the subject to light passing through the product.
